# EUROPEAN PATENT APPLICATION

(11) **EP 1 974 734 A1**
(43) Date of publication of application: **01.10.2008**
(21) Application number: 07105072.8
(22) Date of filing: 28.03.2007
(51) Int. Cl.: A61K 35/74, A61P 3/04

(54) **Probiotics for reduction of risk of obesity**

(71) Applicant: Nestec S.A., 1800 Vevey (CH)
(72) Inventor: The designation of the inventor has not yet been filed
(74) Representative: Dixon, Sarah

(57) **Abstract**

The use of probiotic bacteria capable of promoting the development of an early bifidogenic intestinal microbiota in the manufacture of a medicament or therapeutic nutritional composition for reducing the risk of development of overweight or obesity of an infant later in life.

## Description

### Field of the Invention

This invention relates to the pre-and/or post-natal administration to an infant of probiotic bacteria capable of promoting an early bifidogenic gut microflora with the intention of reducing the risk of the infant developing obesity later in life.

### Background to the Invention

The prevalence of obesity and overweight in adults, children and adolescents has increased rapidly over the past 30 years in the United States and globally and continues to rise. Overweight and obesity are classically defined based on the percentage of body fat or, more recently, the body mass index or BMI. The BMI is defined as the ratio of weight in Kg divided by the height in metres, squared. As overweight and obesity become more prevalent in all age groups, it is inevitable that the number of women giving birth who are also overweight or obese will increase. It is known that overweight and obese women who become pregnant have a greater risk of developing gestational diabetes. Maternal hyperglycaemia may lead to infants with increased body size and fat mass and such infants are themselves prone to develop obesity and diabetes later in childhood or in adult life. Moreover, recent research has suggested that obese women who themselves have normal glucose tolerance give birth to infants with a higher fat mass than those born to women who are not obese.

An increasing amount of scientific evidence suggests that infants born to overweight and obese mothers have a greater risk of becoming overweight or obese later in life than infants born to mothers who are not overweight or obese. This predisposition appears to be higher if both parents are affected. Childhood overweight and obesity currently affects 18 million children under age 5 worldwide. Almost 30% of US children and adolescents and between 10 and 30% of European children are overweight or obese.

Obesity is generally seen as resulting from a combination of excessive energy intake with a sedentary lifestyle. Clearly, these factors are important. More recently, however, it has been suggested that systemic low-grade inflammation and a sub-optimal gut microbiota may also be implicated (Fantuzzi G. "Adipose tissue, adipokines, and inflammation" J Allergy Clin Immunol. 2005;115:911-919,. Bäckhed F, Ding H, Wang T, et al. "The gut microbiota as an environmental factor that regulates fat storage" Proc Natl Acad Sci USA. 2004;101:15718-15723).

Recent meta-analyses have concluded that having been breast-fed is associated with a 13-22% reduced likelihood of overweight or obesity in childhood and that the duration of breast-feeding is inversely associated with the risk of overweight (Owen CG, Martin RM, Whincup PH, Smith GD, Cook DG. "Effect of infant feeding on the risk of obesity across the life course: a quantitative review of published evidence" Pediatrics. 2005;115:1367-1377, Arenz S, Ruckerl R, Koletzko B, von Kries R. "Breast-feeding and childhood obesity: a systemic review" Int J obes Relat Metab Disord. 2004;28:1247-1256, Harder T, Bergmann R, Kallischnigg G, Plagemann A. "Duration of breastfeeding and risk of overweight: a meta-analysis" Am J Epidemiol. 2005;162:397-403).

There is clearly a need to provide methods to address the risk of overweight and obesity, particularly during childhood.

### Summary of the Invention

Change in intestinal microbiota particularly during the critical maturational period of early infancy have already been linked to the development of inflammatory conditions such as allergy. A possible relationship between obesity and asthma has also been suggested. Together, these considerations led the present inventors to investigate the possibility of a relationship between intestinal microbiota in infants and the later weight-gain of those infants.

During a prospective follow-up study on probiotics in allergic disease (described in more detail in Kalliomäki et al., "Probiotics in primary prevention of atopic disease: a randomised placebo-controlled trial", Lancet 2001;357:1076 - 1079), the present inventors have surprisingly found that the weight and body mass index at age 4 of children who received the probiotics are lower than those of children who received a placebo.

Accordingly, in a first aspect the present invention provides the use of probiotic bacteria capable of promoting the development of an early bifidogenic intestinal microbiota in the manufacture of a medicament or therapeutic nutritional composition for reducing the risk of development of overweight or obesity of an infant later in life.

The invention extends to a method of reducing the risk of an infant developing obesity later in life by providing to an infant in need thereof probiotic bacteria capable of promoting the development of an early bifidogenic intestinal microbiota.

Without wishing to be bound by theory, the inventors believe that differences, deviations and/or aberrancies in the intestinal microbiota, particularly as regards the proportion of Bifidobacteria which are present may precede the development of overweight and obesity. Specifically, the establishment of an early, strongly bifidogenic microbiota may provide protection against the later development of overweight and obesity. It should be noted that, in the breast-fed infant, Bifidobacteria form the basis of the microbiota accounting for 60-90 % of total bacteria in the infant gut. Breast feeding also promotes intestinal barrier development which, together with bifidobacterial domination leads to enhanced absorption and therefore utilisation of ingested nutrition.

The intestinal microbiota plays an important role in the hydrolysis of indigestible oligosaccharides and polysaccharides to absorbable monosaccharides and activation of lipoprotein lipase by direct action on the villous epithelium. Further, it has recently been demonstrated that human milk contains not only oligosaccharides but also Bifidobacteria. At the same time, genomic studies have convincingly shown that Bifidobacteria present in the gut of breast-fed infants, such as *Bifidobacterium longum,* are specially equipped to utilize breast-milk oligosaccharides as nutrients. *Bifidobacterium longum* is also adapted to the conditions in the large intestine where energy harvest from slowly absorbable carbohydrates takes place.

### Detailed Description of the Invention

In this specification, the following terms have the following meanings:-
"body mass index " or "BMI" means the ratio of weight in Kg divided by the height in metres, squared.
"early bifidogenic intestinal microbiota" means for infants up to the age of 12 months an intestinal microbiota which is dominated by Bifidobacteria such as *Bifidobacterium breve, Bifidobacterium infantis,* and *Bifidobacterium longum* to the exclusion of appreciable populations of such species as Clostridia and Streptococci and which is generally comparable with that found in breast fed infants.
"infant" means a child under the age of 12 months.
"overweight" is defined for an adult as having a BMI between 25 and 30
"obese" is defined for an adult as having a BMI greater than 30
"probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

All references to percentages are percentages by weight unless otherwise stated.

The probiotic bacteria capable of promoting the development of an early bifidogenic intestinal microbiota are administered to the infant at least during the first two months of the life of the infant. Preferably, they are also administered to the pregnant woman for at least two weeks before delivery and after delivery to the newborn infant for at least two months. After delivery, administration may be either via the breast feeding mother or directly to the new-born infant.

The probiotic bacteria may be any lactic acid bacteria or Bifidobacteria with established probiotic characteristics which are also capable of promoting the development of an early bifidogenic intestinal microbiota. Suitable probiotic lactic acid bacteria include *Lactobacillus rhamnosus* ATCC 53103 obtainable inter alia from Valio Oy of Finland under the trade mark LGG and *Lactobacillus rhamnosus* CGMCC 1.3724. Suitable probiotic Bifidobacteria strains include *Bifidobacterium lactis* CNCM I-3446 sold *inter alia* by the Christian Hansen company of Denmark under the trade mark Bb12, *Bifidobacterium longum* ATCC BAA-999 sold by Morinaga Milk Industry Co. Ltd. of Japan under the trade mark BB536, the strain of *Bifidobacterium breve* sold by Danisco under the trade mark Bb-03, the strain of *Bifidobacterium breve* sold by Morinaga under the trade mark M-16V and the strain of *Bifidobacterium breve* sold by Institut Rosell (Lallemand) under the trade mark R0070. A mixture of suitable probiotic lactic acid bacteria and Bifidobacteria may be used.

A suitable daily dose of the probiotic bacteria is from 10e5 to 10e11 colony forming units (cfu), more preferably from 10e7 to 10e10 cfu.

The probiotic bacteria may be administered to both the pregnant woman before birth and to the mother after birth as a supplement in the form of tablets, capsules, pastilles, chewing gum or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The supplement may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. In all cases, such further components will be selected having regard to their suitability for the intended recipient.

Alternatively, the probiotic bacteria may be administered to pregnant women in the form of a therapeutic nutritional composition. The composition may be a nutritionally complete formula.

A nutritionally complete formula for administration to pregnant women according to the invention may comprise a source of protein. Any suitable dietary protein may be used for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred. The composition may also contain a source of carbohydrates and a source of fat.

If the formula includes a fat source in addition to the DHA, the fat source preferably provides 5% to 40% of the energy of the formula; for example 20% to 30% of the energy. A suitable fat profile may be obtained using a blend of canola oil, corn oil and high-oleic acid sunflower oil.

A source of carbohydrate may be added to the formula. It preferably provides 40% to 80% of the energy of the formula. Any suitable carbohydrate may be used, for example sucrose, lactose, glucose, fructose, corn syrup solids, maltodextrins, and mixtures thereof. Dietary fibre may also be added if desired. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, gum Arabic, fructooligosaccharides, galacto-oligosaccharides, sialyl-lactose and oligosaccharides derived from animal milks. A preferred fibre blend is a mixture of inulin with shorter chain fructo-oligosaccharides. Preferably, if fibre is present, the fibre content is between 2 and 40 g/l of the formula as consumed, more preferably between 4 and 10 g/l.

The formula may also contain minerals and micronutrients such as trace elements and vitamins in accordance with the recommendations of Government bodies such as the USRDA. For example, the formula may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B12, 100 to 800 µg folic acid, 30 to 70 µg biotin, 1 to 5 µg Vitamin D, 3 to 10 IU Vitamin E.

One or more food grade emulsifiers may be incorporated into the formula if desired; for example diacetyl tartaric acid esters of mono- and di- glycerides, lecithin and mono- and di-glycerides. Similarly suitable salts and stabilisers may be included.

The formula is preferably enterally administrable; for example in the form of a powder for re-constitution with milk or water.

The probiotic bacteria may be conveniently administered to infants in an infant formula. An infant formula for use according to the present invention may contain a protein source in an amount of not more than 2.0 g/100kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The infant formula may contain a carbohydrate source. Any carbohydrate source conventionally found in infant formulae such as lactose, saccharose, maltodextrin, starch and mixtures thereof may be used although the preferred source of carbohydrates is lactose. Preferably the carbohydrate sources contribute between 35 and 65% of the total energy of the formula.

The infant formula may contain a source of lipids. The lipid source may be any lipid or fat which is suitable for use in infant formulas. Preferred fat sources include palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5: 1 to about 15:1; for example about 8 : 1 to about 10:1.

The infant formula may also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary, the infant formula may contain emulsifiers and stabilisers such as soy lecithin, citric acid esters of mono- and di-glycerides, and the like.

The infant formula may optionally contain other substances which may have a beneficial effect such as fibres, lactoferrin, nucleotides, nucleosides, and the like.

Both the infant formula and the nutritional formula described above may be prepared in any suitable manner. For example, they may be prepared by blending together the protein, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture. The liquid mixture is then homogenised; for example in two stages.

The liquid mixture may then be thermally treated to reduce bacterial loads, by rapidly heating the liquid mixture to a temperature in the range of about 80°C to about 150°C for about 5 seconds to about 5 minutes, for example. This may be carried out by steam injection, autoclave or by heat exchanger; for example a plate heat exchanger.

Then, the liquid mixture may be cooled to about 60°C to about 85°C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

The homogenised mixture is transferred to a suitable drying apparatus such as a spray drier or freeze drier and converted to powder. The powder should have a moisture content of less than about 5% by weight.

The selected probiotic bacteria may be cultured according to any suitable method and prepared for addition to the nutritional or infant formula by freeze-drying or spray-drying for example. Alternatively, bacterial preparations can be bought from specialist suppliers such as Christian Hansen and Valio already prepared in a suitable form for addition to food products such as nutritional and infant formulas. The probiotic bacteria may be added to the formula in an amount between 10e3 and 10e12 cfu/g powder, more preferably between 10e7 and 10e12 cfu/g powder.

The invention will now be further illustrated by reference to the following examples:-

### Example 1

An example of the composition of a suitable infant formula to be used in the present invention is given below

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin Kl (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| *L. rhamnosus* ATCC 53103 | 2.10⁷ cfu/g of powder, live bacteria | |

### Example 2

This example compares the effect of administering *Lactobacillus rhamnosus* ATCC 53103 prenatally to pregnant women and postnatally to the infants for 6 months upon weight and BMI of the children at the age of 4 years with the same measures for mothers and infants who received a placebo in a double-blind, randomised clinical trial.

Families were recruited in ante-natal clinics in the city of Turku, Finland (population 170,000) between February 1997 and January 1998. Altogether 159 women were randomised by means of a computer to receive two capsules of placebo (microcrystalline cellulose) or 10e10 colony forming units of *Lactobacillus rhamnosus* ATCC 53103 once a day for 2 to 4 weeks before delivery. After delivery, breast-feeding mothers has the option of consuming capsules themselves or otherwise the agents were mixed with water and administered to the infants by spoon. Both these modes of administration have been shown to result in comparable amounts of *Lactobacillus rhamnosus* in infant faeces (Majamaa and Isolauri, 1997). Probiotic-containing and placebo capsules looked, smelled and tasted identical. Capsules were consumed for 6 months postnatally. Codes were kept by the supplier until all data were collected and analysed. The study was approved by the Committees on Ethical Practice in Turku University Hospital and the Health Office of the City of Turku. Written informed consent was obtained from the children's parents.

Subjects were examined at birth and at the ages of 3, 6, 12, 18, 24 months and 4 years with weight and height assessment. Body mass index (BMI) at 4 years was calculated using the International Obesity Task Force criteria for overweight and obesity. These criteria identify BMI values for each age associated with a predicted BMI 25 or 30, respectively, at age 18 to avoid under-estimating the extent of adiposity in childhood. Skinfold measurements were taken in the the biceps, triceps, sub-scapular and suprailiac regions and the circumference of the mid upper arm was measured.

### RESULTS

The results are presented in Table 1.

**Table 1. Anthropometric measures in 4 year old children having received probiotics or placebo during perinatal period. Data is presented as mean (SD)¹.**

| | | Probiotics | | Placebo | | P value² |
|---|---|---|---|---|---|---|
| Weight | | | | | | |
| | kg | 17.6 | (1.7) | 18.1 | (2.9) | 0.346 |
| | % for height | 0.0 | (8.5) | 3.4 | (10.8) | 0.075 |
| Height | | | | | | |
| | cm | 106.1 | (3.5) | 105.3 | (5.1) | 0.342 |
| | SD scores | 0.4 | (0.6) | 0.3 | (1.1) | 0.801 |
| BMI | | 15.7 | (1.3) | 16.2 | (1.6) | 0.052 |
| Body fat, % | | 15.5 | (3.6) | 15.8 | (4.2) | 0.679 |
| Skinfolds, mm | | | | | | |
| | Biceps | 5.4 | (1.8) | 5.5 | (1.9) | 0.582 |
| | Triceps | 9.2 | (2.7) | 9.5 | (2.4) | 0.623 |
| | Subscapular | 5.8 | (1.0) | 6.2 | (2.1) | 0.219 |
| | Suprailiac | 4.1 | (1.1) | 4.4 | (1.7) | 0.312 |
| Circumferences, cm | | | | | | |
| | Mid upper arm | 17.6 | (1.5) | 17.4 | (1.5) | 0.633 |
| | Mid upper arm muscle | 14.7 | (1.1) | 14.5 | (1.2) | 0.380 |

| | | | | | | |
|---|---|---|---|---|---|---|
| ¹N 42-53 in placebo and 35-51 in probiotics group. ²Independent samples t-test | | | | | | |

The subjects whose data is presented in Table 1 were divided into two groups, those receiving the probiotic intervention and those receiving a placebo. It may be seen from these results that the mean BMI of the group receiving the intervention is lower that the mean BMI of the placebo group. In addition, other measures of body fat such as the skinfold measurements were consistently smaller for the intervention group.

However, as may be seen from the report of this study published in The Lancet, some subjects in both groups developed atopic diseases. As it is already known that the development of atopic disease may be associated with growth as measured by height and overall weight gain (see, for example Laitinen et al., "Evaluation of diet and growth in children with and without atopic eczema: follow-up study from birth to 4 years", British Journal of Nutrition (2005), 94, 565 - 574), the data was re-evaluated, this time including only measurements from healthy children. The results are shown in Table 2.

**Table 2. Anthropometric measures in 4 year old children having received probiotics or placebo during perinatal period. Only children without atopic eczema are included. Data is presented as mean (SD)¹.**

| | | Probiotics | | Placebo | | P value² |
|---|---|---|---|---|---|---|
| Weight | | | | | | |
| | kg | 17.8 | (1.9) | 18.2 | (3.6) | 0.616 |
| | % for height | 1.6 | (9.3) | 4.4 | (12.7) | 0.301 |
| Height | | | | | | |
| | cm | 105.7 | (3.3 | 104.7 | (6.4) | 0.464 |
| | SD scores | 0.3 | (0.8) | 0.3 | (1.3) | 0.838 |
| BMI | | 15.9 | (1.5) | 16.4 | (1.9) | 0.221 |
| Body fat, % | | 16.1 | (3.0) | 16.9 | (4.6) | 0.526 |
| Skinfolds, mm | | | | | | |
| | Biceps | 5.7 | (2.1) | 6.2 | (2.4) | 0.445 |
| | Triceps | 9.5 | (2.8) | 10.0 | (2.7) | 0.482 |
| | Subscapular | 5.8 | (1.0) | 6.7 | (2.6) | 0.088 |
| | Suprailiac | 4.1 | (1.0) | 4.9 | (2.1) | 0.119 |
| Circumferences, cm | | | | | | |
| | Mid upper arm | 17.9 | (1.5) | 17.6 | (1.9) | 0.538 |
| | Mid upper arm muscle | 15.0 | (1.1) | 14.5 | (1.1) | 0.236 |

| | | | | | | |
|---|---|---|---|---|---|---|
| ¹ N 21-28 in placebo and 23-36 in probiotics group. ²Independent samples t-test | | | | | | |

From Table 2, it may be seen that also for healthy children both the mean BMI of subjects receiving the intervention as well as such other measures of body fat as the skinfold measurements are consistently lower that the corresponding measurements for subjects not receiving the intervention.

## Claims

1. The use of probiotic bacteria capable of promoting the development of an early bifidogenic intestinal microbiota in the manufacture of a medicament or therapeutic nutritional composition for reducing the risk of development of overweight or obesity of an infant later in life.

2. The use of Claim 1, wherein the probiotic bacteria are lactic acid bacteria.

3. The use of Claim 2, wherein the lactic acid bacteria are of the strain *Lactobacillus rhamnosus* ATCC 53103 or *Lactobacillus rhamnosus* CGMCC 1.3724.

4. The use of Claim 1, wherein the probiotic bacteria are Bifidobacteria.

5. The use of Claim 4, wherein the Bifidobacteria are of the strain *Bifidobacterium lactis* CNCM I-3446, *Bifidobacterium longum* ATCC BAA-999, *Bifidobacterium breve* Bb-03, *Bifidobacterium breve* M-16V or *Bifidobacterium breve* R0070.

6. The use of any preceding claim wherein the medicament or nutritional composition is administered to a pregnant woman for at least two weeks before delivery and, after delivery, to the infant for at least 2 months.

7. The use of any preceding claim, wherein the medicament or nutritional composition is administered to the infant for at least 6 months after delivery.

8. The use of Claim 6 or 7, wherein, after delivery, the probiotic bacteria are administered to the infant via the breast-feeding mother.

9. The use of any of Claims 1 to 7, wherein the therapeutic nutritional composition is an infant formula.

10. The use of any preceding claim wherein the medicament comprises between 10e5 and 10e10 cfu of probiotic bacteria per daily dose.

11. The use of any preceding claim wherein the therapeutic nutritional composition comprises between 10e3 and 10e12 cfu/g of composition (dry weight).
